# EUROPEAN PATENT SPECIFICATION

(11) **EP 1 414 428 B1**
(45) Date of publication and mention of the grant of the patent: **01.07.2009**
(21) Application number: 02764796.5
(22) Date of filing: 26.07.2002
(51) Int. Cl.: A61K 31/166, A61P 19/02, A61P 25/02, A61P 29/00, A61P 1/04, A61P 9/10, A61P 11/00, A61P 11/06, A61K 31/165

(54) **HISTONE DEACETYLASE ENZYME-INHIBITING DERIVATIVES OF HYDROXAMIC ACID AS NEW CYTOKINE SYNTHESIS-INHIBITING ANTI-INFLAMMATORY DRUGS**
HISTON DEAZETYLASE ENZYM-HEMMENDE HYDROXAMSÄUREDERIVATE ALS NEUE ENTZÜNDUNGSHEMMER ZUR HEMMUNG DER ZYTOKINSYNTHESE
DERIVES DE L'ACIDE HYDROXAMIQUE INHIBITEURS DE L'ENZYME HISTONE DEACETYLASE UTILISES COMME NOUVEAUX MEDICAMENTS ANTI-INFLAMMATOIRES INHIBITEURS DE LA SYNTHESE DE LA CYTOKINE

(30) Priority: 07.08.2001 IT MI20011733
(43) Date of publication of application: 06.05.2004
(73) Proprietor: ITALFARMACO S.p.A., 20126 Milano (IT)
(72) Inventor: MASCAGNI, Paolo, I-20092 Cinisello Balsamo (IT); LEONI, Flavio, I-20092 Cinisello Balsamo (IT); PORRO, Giulia, I-20092 Cinisello Balsamo (IT); PAGANI, Paolo, I-20092 Cinisello Balsamo (IT); DONA', Giancarlo, I-20092 Cinisello Balsamo (IT); POZZI, Pietro, I-20092 Cinisello Balsamo (IT); DINARELLO, Charles, I-20092 Cinisello Balsamo (IT); FANTUZZI, Giamila, I-20092 Cinisello Balsamo (IT); SIEGMUND, Britta, I-20092 Cinisello Balsamo (IT); REZNIKOV, Leonid, I-20092 Cinisello Balsamo (IT); BUFLER, Philip, I-20092 Cinisello Balsamo (IT); KIM, Soo-Hyun, I-20092 Cinisello Balsamo (IT); POMERANTZ, Benjamin, 20094 Cinisello Balsamo (IT)
(74) Representative: Pistolesi, Roberto
(86) International application number: PCT/EP2002/008379
(87) International publication number: WO 2003/013493

(56) References cited:
- EP-B- 0 574 758
- EP-B- 0 757 984
- WO-A-97/35990
- WO-A-97/43251
- WO-A-02/055017
- HUANG N ET AL: "INHIBITION OF IL-8 GENE EXPRESSION IN CACO-2 CELLS BY COMPOUNDS WHICH INDUCE HISTONE HYPERACETYLATION" CYTOKINE, ACADEMIC PRESS LTD, PHILADELPHIA, PA, US, vol. 9, no. 1, January 1997 (1997-01), pages 27-36, XP001013211 ISSN: 1043-4666
- MISHRA NILAMADHAB ET AL: "Histone deacetylase inhibitor Trichostatin A as a strong candidate for treatment of systemic lupus erythematosus." FASEB JOURNAL, vol. 15, no. 5, 8 March 2001 (2001-03-08), page A1214 XP009002039 Annual Meeting of the Federation of American Societies for Experimental Biology on Experimental Biology 2001;Orlando, Florida, USA; March 31-April 04, 2001 ISSN: 0892-6638
- RICHON VICTORIA M ET AL: "A class of hybrid polar inducers of transformed cell differentiation inhibits histone deacetylases." PROCEEDINGS OF THE NATIONAL ACADEMY OF SCIENCES OF THE UNITED STATES, vol. 95, no. 6, 17 March 1998 (1998-03-17), pages 3003-3007, XP001120542 March 17, 1998 ISSN: 0027-8424

## Description

This invention relates to the use of hydroxamic acid derivatives having histone deacetylase enzyme-inhibiting activity for the preparation of anti-inflammatory medicaments.

Some derivatives of hydroxamic acid which inhibit histone deacetylases are known. Those which have been most extensively studied are suberoylanilide hydroxamic acid (SAHA), N-hydroxy-3-[3-(hydroxyamino)-3-oxo-1-propenyl]-benzamide (CBHA) and trichostatin (TSA). Other derivatives are described in Proc: Natal. Acad. Sci. USA 95, 3003-3007, 1998; Tumori, 2001 Nov-Dec, 87 (6): S12-4; Anticancer Drugs, 2002 Jan, 13 (1): 1-13; Nature Rev Cancer, 2001 Dec, 1 (3): 194-202; Curr Opin Oncol, 2001 Non. 13 (6): 477-83; Cancer Chemother Pharmacol, 2001, Aug, 48 Suppl 1:S20-6; Cancer Chemother Pharmacol, 2001 Aug, 48 Suppl 1:S17-9; Haematologica, 2001 Sep; 86 (9): 908-17.

These compounds have mainly been studied as potential anti-tumoral drugs: trichostatin, an antifungal antibiotic isolated from *Streptomyces hygroscopicus,* is a potent inducer of murine erythroleukaemic cell differentiation (Cancer Res. 47, 3288-3691, 1987), while SAHA and CBHA have been studied by the Sloan Lettering Institute (WO 95/31977) as tumour cell differentiation inducing agents.

The therapeutic use of histone deacetylase inhibitors to treat tumours is described in Anticancer Res. 20, 1471-1486, 2000 and Exp.Opin.Invest. Drugs 8(10),1611-1621,1999.

It has now been found that the known derivatives of hydroxamic acid having histone deacetylase inhibiting activity, especially SAHA, inhibit the synthesis of pro-inflammatory cytokines, and can therefore be used to treat disorders which can be alleviated by inhibiting those cytokines. Examples of such disorders, with an inflammatory and/or autoimmune basis, include multiple sclerosis, Crohn's disease and atherosclerosis, rheumatoid arthritis, spondyloarthropathies (anchilosating spondilitis, psoriatic arthritis, arthritis connected to ulcerative colitis), AIDS-related neuropathies, asthma, chronic obstructive lung diseases, bronchitis, pleuritis, acute and chronic hepatitis (either viral, bacterial or toxic), and, broadly speaking, all disorders with an inflammatory component

For the therapeutic uses considered, the hydroxamic acid derivatives will be administered at doses ranging between 1 and 500 mg one or more times a day, depending on the disorder concerned and the pharmacotoxicological characteristics of the compound in question, which can be administered in the form of suitable oral, parenteral or topical formulations.

The following examples illustrate the invention in greater detail.

### EXAMPLE 1- Inhibition of cytokine production in vitro

The treatment of lymphocytes with lipopolysaccharide (LPS) induces the production of various pro-inflammatory cytokines, such as TNFα, IL-1β and IFNγ (J. Biol. Chem. 1990; 265(18): 10232-10237; Science, 1998; 281:1001-1005).

The effect of SAHA has been studied by evaluating the inhibitory effect of the compound on cytokine production by peripheral blood mononuclear cells (PBMC) from healthy volunteers (2 to 6 donors), stimulated with LPS.

Samples of peripheral blood or buffy coats from healthy volunteers were used. The samples were separated by centrifugation on density gradient using Ficoll-Hypaque, and the PBMCs thus obtained were seeded in 96-well dishes (500,000 cells/well), incubated for 60 minutes with SAHA at various doses, and then stimulated with LPS from *E*. *coli* 055:B5 (10 ng/ml) for 24 hours in the presence of the compound. At the end of the treatment the pro-inflammatory cytokines TNFα, IL-1β were measured by means of an electrochemiluminescence assay (ECL) using specific commercial antibodies.

Interferon γ (IFNγ) was measured with a commercially available ELISA assay.

Cytokine IFNγ is produced by the T lymphocytes following their stimulation by pro-inflammatory cytokines, especially IL-12 and IL-18 (Dinarello C. A. and Moldawer L. L. Proinflammatory and anti-inflammatory cytokines in rheumatoid arthritis. A primer for clinicians. 2nd Edition, Amgen Inc., 2000).

The effect of SAHA on IFNγ synthesis induced by stimulating PBMCs with IL-12 and IL-18 *in vitro* was evaluated on this basis. PBMCs were seeded in round-bottomed 96-well dishes (500,000 cells/dish), and incubated with various doses of SAHA for 60 minutes. At the end, the cells were stimulated for 48 hours in the presence of the compound by simultaneous addition of recombinant IL-12 (10 ng/ml) and recombinant IL-18 (20 ng/ml). The quantity of IFNγ produced was determined with a commercial ELISA assay.

The effect of the various doses of SAHA was measured as the percentage inhibition of production of the cytokine in question compared with untreated control cells. The concentration able to induce 50% inhibition of cell growth (IC₅₀) was determined by linear regression.
The results are summarised in the table below:

| **Cytokine** | **SAHA IC₅₀ (nM)** | **TSA IC₅₀ (nM)** |
|---|---|---|
| TNFα | 200 | 50 |
| IL-1β | 100 | 100 |
| IFNγ | 50 | 10 |
| IFNγ (from IL-12 + IL-18) | 740 | 490 |

These results clearly indicate that SAHA inhibit synthesis of all the inflammatory cytokines induced by LIPS with an IC₅₀ in the nanomolar range (50-200nM).

SAHA also inhibit the synthesis of IFNγ by the T lymphocyte cells, as demonstrated by their efficacy (IC₅₀ 740 nM and 490 nM respectively) when the stimulus used was the combination of IL-12 and IL-18 specific for that cell line.

### EXAMPLE 2 - Inhibition of cytokine production in vivo

Systemic administration of LPS to laboratory animals is known to induce rapid, massive production of pro-inflammatory cytokines (Immunopharmacol. 1992; 14(6): 1045-1050).

Female BALB/c mice (20-22 grams) were treated orally with SAHA at the various doses indicated, then treated after 60 minutes with LPS from E. *Coli* 055:B5 (30 mg/Kg intraperitoneally). 90 minutes after the endotoxin administration, blood samples were taken from all the treated animals (10 animals/group), and the cytokines were measured with commercial ELISA assays.

The results are set out in the table below, and expressed as the percentage inhibition of production of the cytokine in question:

| **TREATMENT** | **% inhibition of TNFα** | **Inhibition of IL-1β** | **Inhibition of IL-6** |
|---|---|---|---|
| SAHA | | | |
| 0.1 mg/Kg | 40 | 13 | 10 |
| 1 mg/Kg | 53 | 15 | 3 |
| 10 mg/Kg | 67 | 3 5 | 7 |
| 25 mg/Kg | 68 | 37 | 25 |
| 50 mg/Kg | not done | 51 | 29 |

The above results indicate that SAHA is active orally and able to inhibit, to a dose-dependent extent, pro-inflammatory cytokine synthesis induced in *vivo* in the mouse by administering endotoxin, thus confirming the results *obtained in vitro.*

### EXAMPLE 3. Con A-Induced Liver Injury.

C57B16 mice were injected i.p. with either water vehicle or SAHA and after 1 h were injected i.v. with Con A as described in Proc. Natl. Acad. Sci. USA, (2000), 97, 2367-2372. After 24 h, serum amino-alanine transferase was measured.

Intravenous injection of Con A results in hepatic cell death within 12-24 h with markedly elevated serum levels of hepatic enzymes such as alanine amino transaminase (ALT). In mice pretreated with a single dose of SAHA (50 mg/kg) given i.p. 1 h before Con A, the 24-h level of serum ALT (mean ± SE) was 8.144 ± 2.091 units/liter compared with 15.190 ± 2.580 in vehicle-treated mice (n =6 per group).

### EXAMPLE 4. Nitric Oxide Production from Mouse Peritoneal Macrophages.

C57BLy6 mice were injected i.p. with 1 ml of sterile thioglycolate broth and killed after 5 days, and macrophages were isolated using instillation of 10 ml of ice-cold PBS into the peritoneal cavity. The cells were centrifuged (350 x g) and 3 ml of erythrocyte lysing reagent (PharMingen) was added for 10 min.

Seven milliliters of DMEM containing 5% FCS was added and the cells were centrifuged at 4°C. The cells were resuspended in DMEM at 1 million per ml and 0.5 ml were added to wells of a 48-well plate. SAHA was added for 60 min at 37°C and then stimulated with the combination of TNF-α plus IFN-γ. After 24 h, NO levels in the supernatant were determined using the Griess reagent as described in Am.J.Physiol.Cell Physiol. (2001), 280, C441-C450.

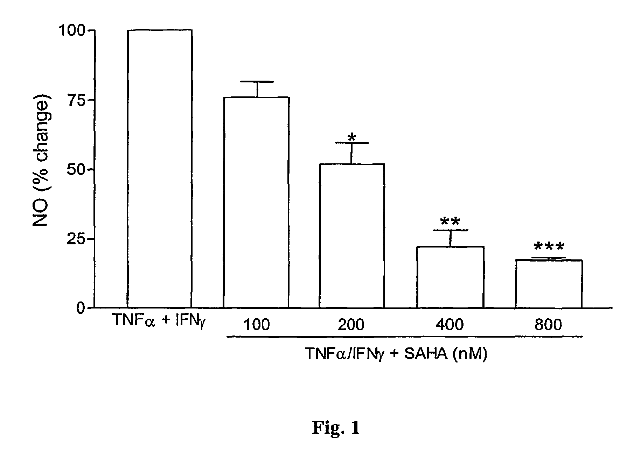

As shown in Fig. 1, SAHA inhibited NO production; at 200 nM, there was a 50% reduction (P, 0.05). Further reductions of 80 and 85% were observed at 400 and 800 nM, respectively.

### EXAMPLE 5. Inhibition of IL-12 production by cultured monocytes.

Venous blood was obtained from consenting adults and separated over Ficoll-Hypaque. The PBMC fraction was washed and adjusted to five million cells per ml. Five hundred microliters was aliquoted into each well of 24-well flat-bottom plates, 100 ml of SAHA was added, and the plates were incubated for 1 h at 37°C. The cells were stimulated with LPS, soluble OKT3, or cytokines, and after 24 or 48 h At 37°C the supernatant was removed and frozen for cytokine assays. Monocytes were isolated by centrifugation over Percoll, washed, suspended in RPMI with 10% FCS, and aliquoted at 2 million cells per ml in Petriperm Teflon-coated culture dishes (Sigma). The ELISA for human IL-12 (p70) was purchased from Endogen (Woburn, MA).

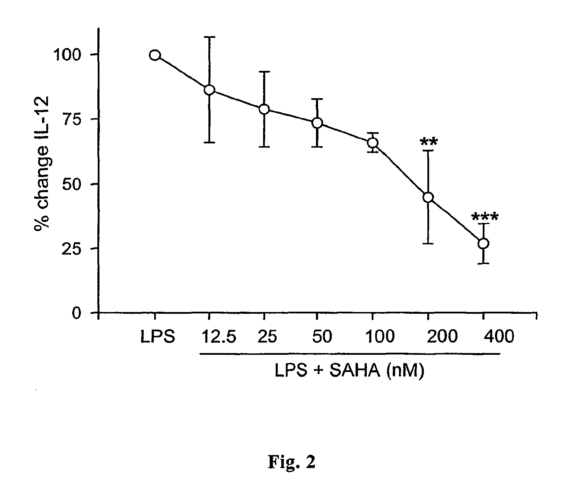

As shown in Fig. 2, there was a dose-dependent reduction in LPS/IFN-γ-induced IL-12 production in nonadherent human monocytes. At 200 nM, the reduction was 55% *(P,* 0.01) and at 86% at 400 nM (P, 0.001).

### EXAMPLE 6 Dextran-induced colitis

Female, 8 week-old C57BL/6 mice (The Jackson Laboratories, Bar Habor, ME) weighing 20-22 g were used in this study. The animals were housed in rooms at a controlled temperature and a 12 h day/night rhythm. They were fed standard mice chow pellets ad libitum, had free access to tap water supplied in bottles, and were acclimatized to the conditions at least seven days before they were used in experiments. Mice were killed by cervical dislocation under isoflurane anesthesia (Fort Dodge, Iowa City, IA).

Mice were fed 3.5% dextran sulfate sodium (DSS; molecular weight 40 kDa; ICN, Aurora, OH) dissolved in sterile, distilled water ad libitum from day one to five followed by a five day observation period. SAHA was administered once daily orally (p. o.) in a total volume of 200 µl and a concentration of 10 mg/kg body weight (BW) throughout the experiment (day 1 to 10). Control mice had free access to water and received either SAHA (10 mg/kg BW) or water p. o. once daily for 10 days.

Body weights, occult blood or the presence of gross blood per rectum, and stool consistency were determined daily. Two investigators blinded to the protocol assessed the clinical score (table 1). Weight loss < 1% compared to day 1 was counted as 0 points, weight loss of 1 to < 5% as 1 point, 5 to < 9.9% as 2 points, 10 to 20% as 3 points and more than 20% as 4 points. For stool consistency, 0 points were given for well-formed pellets (formed), 2 points for pasty and semi-formed stools which did not stick to the anus (soft), and 4 points for liquid stools that did stick to the anus (diarrhea). Bleeding was scored 0 points for no blood in hemoccult, 2 points for positive hemoccult, and 4 points for gross bleeding. These scores (body weight, stool consistency, rectal bleeding) were added and divided by 3 resulting in a total clinical score ranging from 0 (healthy) to 4 (maximal activity of colitis).

*Post mortem* (on day 10), the entire colon was removed from the caecum to the anus and the colon length was measured as an indirect marker of inflammation. Colon length has been shown to be a reliable parameter in this model as DSS-induced colitis is associated with colon shortening as described previously [Gastroenterology,1990, 98,:694:J. Pharmacol.Exp.Ther., 2001, 296:99-105].

From the results, reported in the following Tables 1-6, it is evident that SAHA effectively counteracts dextran-induced colitis, a valid and established model of inflammatory bowel diseases in humans.

**Table 1. Clinical activity score [Lab. Invest., 1993, 69:238-249].**

| **Score points** | **Weight loss** | **Stool consistency** | **Rectal bleeding** |
|---|---|---|---|
| 0 | 0% | Formed | Negative hemoccult |
| 1 | (>0%) <5% | | |
| 2 | 5-9.9% | Soft | Positive hemoccult |
| 3 | 10-20% | | |
| 4 | >20% | Diarrhea | Macroscopic bleeding |

**Table 2. Weight**

| | **Days** | | | | | | | | | |
|---|---|---|---|---|---|---|---|---|---|---|
| **Group** | **1** | **2** | **3** | **4** | **5** | **6** | **7** | **8** | **9** | **10** |
| **DSS + SAHA** | 19.1±0.2 | 18.8±0.2 | 19.1±0.3 | 18.4±0.4 | 18.4±0.4 | 18.7±0.3 | 18.0±0.4 | 16.9±0.3 | 16.6±0.4 | 16.6±0.4 |
| **DSS + water** | 18.7±0.5 | 19.0±0.5 | 19.4±0.5 | 18.9±0.5 | 18.9±0.5 | 18.8±0.6 | 18.8±0.6 | 16.6±0.5 | 16.8±0.5 | 16.8±0.5 |
| **SAHA** | 18.9±0.3 | 18.8±0.4 | 18.9±0.2 | 18.9±0.3 | 19.0±0.1 | 19.0±0.3 | 18.9±0.1 | 19.0±0.3 | 19.1±0.1 | 19.1±0.2 |
| **Water** | 19,0±0,2 | 19.0±0.3 | 18.9±0.1 | 19.0±0.2 | 19.0±0.4 | 19.0±0.2 | 19.1±0.2 | 19.1±0.1 | 19.1±0.3 | 19.1±0.1 |

**Table 3. Stool consistency as score**

| | **Days** | | | | | | | | | |
|---|---|---|---|---|---|---|---|---|---|---|
| **Group** | **1** | **2** | **3** | **4** | **5** | **6** | **7** | **8** | **9** | **10** |
| **DSS + water** | 0.0 ± 0.0 | 0.0 ± 0.0 | 0.0 ± 0.0 | 0.4 ± 0.4 | 1.2 ± 0.4 | 2.0 ± 0.0 | 2.0 ± 0.0 | 2.0 ± 0.0 | 1.6 ± 0.4 | 1.6 ±0.4 |
| **DSS + SAHA** | 0.0 ±0.0 | 0.0 ±0.0 | 0.0 ±0.0 | 0.0 ±0.0 | 0.8 ±0.4 | 0.8 ±0.4 | 2.0 ±0.0 | 2.0 ±0.0 | 1.2 ±0.4 | 1.2 ±0.4 |
| **SAHA** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ± 0.0 |
| **Water** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 |

**Table 4. Bleeding**

| | **Days** | | | | | | | | | |
|---|---|---|---|---|---|---|---|---|---|---|
| **Group** | **1** | **2** | **3** | **4** | **5** | **6** | **7** | **8** | **9** | **10** |
| **DSS + water** | 0.0 ± 0.0 | 0.0 ± 0.0 | 0.0 ± 0.0 | 0.8 ± 0.4 | 1.6 ± 0.4 | 2.8 ± 0.4 | 2.0 ± 0.0 | 2.0 ±0.0 | 2.0 ± 0.0 | 1.6 ±0.4 |
| **DSS + SAHA** | 0.0 ±0.0 | 0.0 ±0.0 | 0.0 ±0.0 | 0.0 ±0.0 | 1.2 ±0.4 | 2.4 ±0.4 | 2.0 ±0.0 | 1.6 ±0.4 | 1.2 ±0.4 | 0.8 ±0.4 |
| **SAHA** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 |
| **Water** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 |

**Table 5. Complete clinical score**

| | **Days** | | | | | | | | | |
|---|---|---|---|---|---|---|---|---|---|---|
| **Group** | **1** | **2** | **3** | **4** | **5** | **6** | **7** | **8** | **9** | **10** |
| **DSS+water** | 0.0±0.0 | 0.3±0.1 | 0.2 ±0.1 | 0.8 ±0.1 | 1.1 ±0.2 | 2.1 ±0.1 | 2.0 ±0.1 | 2.1±0.1 | 2.0±0.1 | 1.9±0.2 |
| **DSS+SAHA** | 0.0 ±0.0 | 0.0 ±0.0 | 0.1 ±0.1 | 0.2 ±0.1 | 0.7 ±0.3 | 1.3 ±0.3 | 1.5 ±0.1 | 1.9 ±0.1 | 1.5 ±0.4 | 1.3 ±0.4 |
| **SAHA** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.1 | 0.0 ±0.0 |
| **Water** | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 | 0.0 ±0.0 |

**Table 6. Colon length**

| **Group** | **Colon length (cm)** |
|---|---|
| DSS + water | 7.6 ±0.2 |
| DSS+SAHA | 9.2 ± 0.2 |
| SAHA | 10.1 ± 0.2 |
| Water | 10.5 ± 0.3 |

### EXAMPLE 7

A series of hydroxamic acid derivatives described in EP 901465 were subjected to the histone deacetylase (HDAC) and TUFα inhibition tests in accordance with the methods descried by Lechner et al., Biochim Biophys. Acta 1996, 1296, 181-188 and Moreira A.L. et al., J. Exp. Med. 1993, 177, 1657-1680 respectively.

The results, set out in the following table, show that a linear correlation exists between the ability of these compounds to inhibit the synthesis of TNFα and their inhibition of HDAC activity.

| | 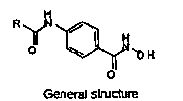 | HDAC | | TNF | |
|---|---|---|---|---|---|
| | R | IC₅₀ nM | Potency | IC₅₀ nM | Potency |
| 1 | 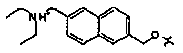 | 20,0 | 100,00 | 7,0 | 100,00 |
| 2 | 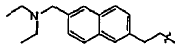 | 62,0 | 32,26 | 10,2 | 68,75 |
| 3 | 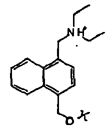 | 65,0 | 30,77 | 10,3 | 68,02 |
| 4 | 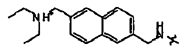 | 78,0 | 25,64 | 11,2 | 67,66 |
| 5 | 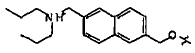 | 46,7 | 42,86 | 12,7 | 55,28 |
| 6 | 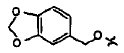 | 80,0 | 25,00 | 50,0 | 14,00 |
| 7 | 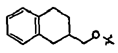 | 91,0 | 21,98 | 65,5 | 10,68 |
| 8 | 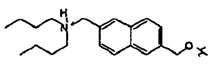 | 133,3 | 15,00 | 67,8 | 10,32 |
| 9 | 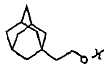 | 600,0 | 3,33 | 159,1 | 4,40 |
| 10 | 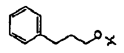 | 105,0 | 19,05 | 159,1 | 4,40 |
| 11 | 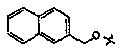 | 8,1 | 246,91 | 159,1 | 4,40 |
| 12 | 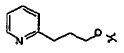 | 260,0 | 7,69 | 230,0 | 3,04 |
| 13 | 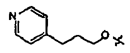 | 260,0 | 7,69 | 270,0 | 2,59 |
| 14 | 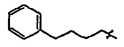 | 86,7 | 23,08 | 300,0 | 2,33 |
| 15 | 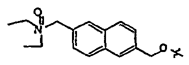 | 206,7 | 9,68 | 1000,0 | 0,70 |

## Claims

1. The use of hydroxamic acid derivatives having histone deacetylase inhibiting activity selected from suberoylanilide hydroxamic acid, N-hydroxy-3[3-(hydroxyamino)-3-oxo-1-propenyl]-benzamide and a compound of formula 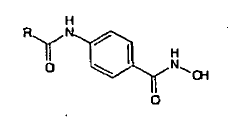 wherein R is selected from: 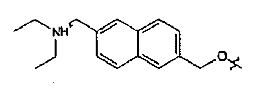 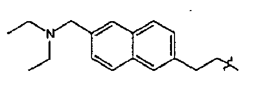 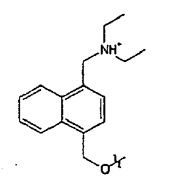 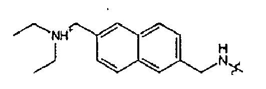 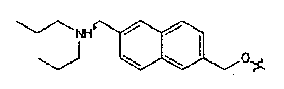 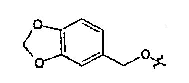 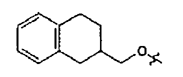 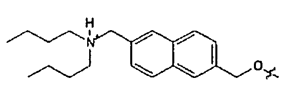 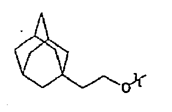 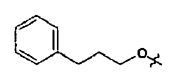 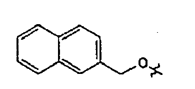 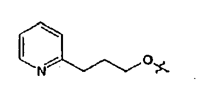 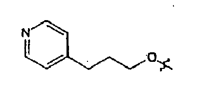 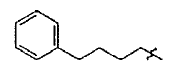 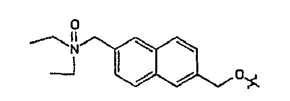 for the preparation of a medicament for the treatment of diseases selected from multiple sclerosis, Crohn's disease, atherosclerosis, rheumatoid arthritis, spondyloarthropathies, AIDS-related neuropathies, asthma, chronic obstructive lung diseases, bronchitis, pleuritis, acute and chronic hepatitis.

2. The use according to claim 1 wherein the hydroxamic acid derivatives are selected from suberoylanilide hydroxamic acid, N-hydroxy-3[3-(hydroxyamino)-3-oxo-1-propenyl]-benzamide.

3. The use according to claims 1 and 2 wherein the medicament is in the form of oral, parenteral or topical formulation.

## Patentansprüche

1. Verwendung von Hydroxamsäurederivaten mit Histon-Deacetylase inhibierender Aktivität, ausgewählt aus Suberoylanilid-Hydroxamsäure, N-Hydroxy-3[3-(hydroxyamino)-3-oxo-1-propenyl]-benzamid und einer Verbindung der Formel 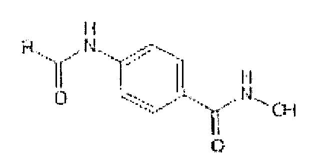 wobei R ausgewählt ist aus: 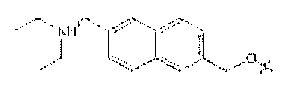 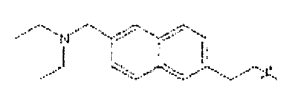 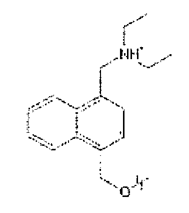 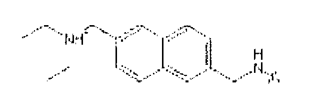 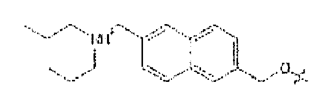 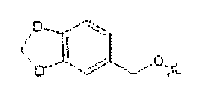 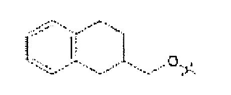 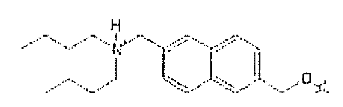 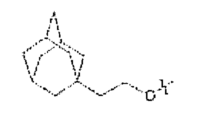 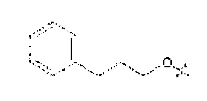 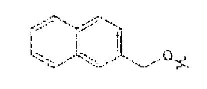 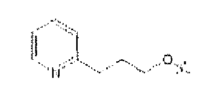 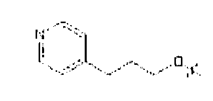 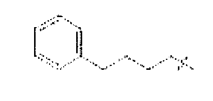 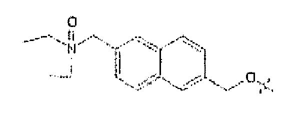 für die Herstellung eines Medikaments zur Behandlung von Krankheiten, ausgewählt aus Multipler Sklerose, Morbus Crohn, Arteriosklerose, rheumatoider Arthritis, Spondyloarthropathien, AIDS-bezogenen Neuropathien, Asthma, chronischen obstruktiven Lungenerkrankungen, Bronchitis, Pleuritis, akuter und chronischer Hepatitis.

2. Verwendung nach Anspruch 1, wobei die Hydroxarnsäurederivate ausgewählt sind aus Suberoylanilid- Hydroxamsäure, N-Hydroxy-3 [3 -(hydroxyamino)-3-oxo-1-propenyl]-benzamid.

3. Verwendung nach den Ansprüchen 1 und 2, wobei das Medikament in Form einer oralen, parenteralen oder topischen Formulierung vorliegt.

## Revendications

1. Utilisation de dérivés de l'acide hydroxamique ayant une activité d'inhibiteur d'histone désacétylase choisis parmi l'acide hydroxamique suberoylanilide, le benzamide N-hydroxy-3[3-(hydroxyammo)-3-oxo-1-propényl] et un compose de formule 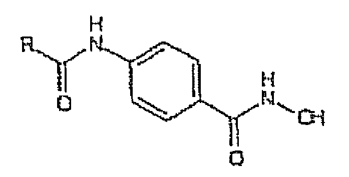 dans lequel R est choisi parmi : 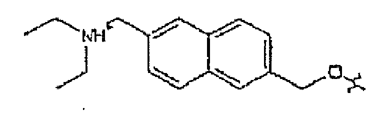 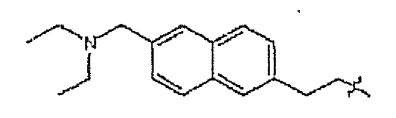 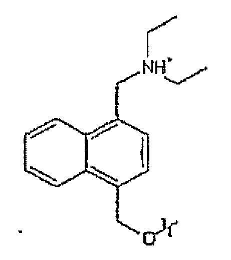 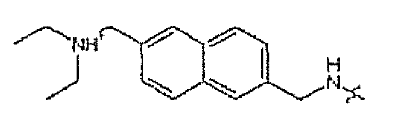 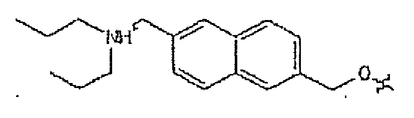 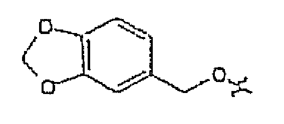 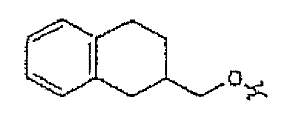 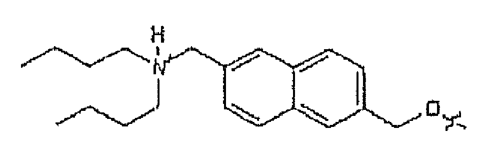 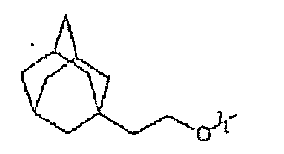 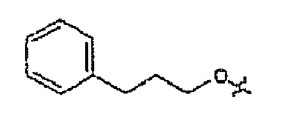 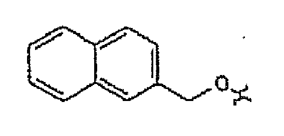 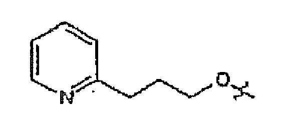 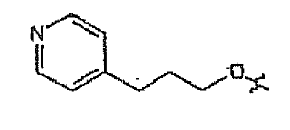 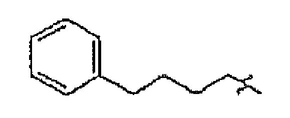 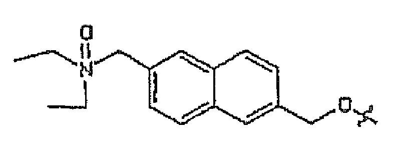 pour la préparation d'un médicament pour le traitement de maladies choisies parmi la sclérose en plaques, la maladie de Crohn, l'athérosclérose, l'arthrite rhumatoïde, les spondylarthropathies, les neuropathies associées au SIDA, l'asthme, les maladies pulmonaires obstructives chronique, les bronchites, les pleurites, les hépatites chroniques et aigües.

2. Utilisation selon la revendication 1, dans laquelle les dérivés de l'acide hydroxamique sont choisis parmi l'acide hydroxamique suberoylanilide et le benzamide N-hydroxy-3 [3-(hydroxyamino)-3-oxo-1-propényl].

3. Utilisation selon les revendications 1 et 2 dans laquelle le médicament est sous forme d'une formulation orale, parentérale ou topique.
